# EUROPEAN PATENT APPLICATION

(11) **EP 1 917 942 A1**
(43) Date of publication of application: **07.05.2008**
(21) Application number: 07254303.6
(22) Date of filing: 30.10.2007
(51) Int. Cl.: A61F 13/15

(54) **Stacked absorbent article assembly**

(30) Priority: 31.10.2006 US 863605 P
(71) Applicant: McNeil-PPC, Inc., Skillman, NJ 08558 (US)
(72) Inventor: Austin, Jennifer J., Trenton NJ 08611 (US); Luizzi, Joseph M., Newtown PA 18940 (US); Tylicki, Jessica G., Ewing NJ 08638 (US)
(74) Representative: Mercer, Christopher Paul

(57) **Abstract**

A stacked absorbent article assembly including a top absorbent article, a bottom absorbent article, an adhesive arranged between the articles for selectively securing the top absorbent article to the bottom absorbent article, and an adhesive arranged on a bottom surface of the assembly for securing the absorbent article assembly to an undergarment.

## Description

### FIELD OF THE INVENTION

This invention relates to disposable absorbent articles, and more particularly to a disposable absorbent article assembly including a plurality of disposable absorbent articles arranged in a stacked configuration, each one of the absorbent articles being adhered to an adjacent absorbent article such that it is selectively removable therefrom.

### BACKGROUND OF THE INVENTION

A number of sanitary articles have been developed for absorbing and containing body fluids produced during menstruation, e.g. sanitary napkins and liners. Sanitary napkins and liners are conventionally constructed to include a liquid pervious body facing cover and a liquid impervious garment facing barrier. Conventional sanitary articles may also include an absorbent core arranged between the cover layer and the barrier layer. Typically the garment facing surface of the barrier layer includes an adhesive for securing the sanitary article to an undergarment during use.

One problem associated with conventional absorbent articles of the type described above is that once the article absorbs body fluid during use, the user may experience discomfort due to a wet feeling against the skin caused by the wet surface of the absorbent article being in abutment with the skin. In an attempt to overcome this shortcoming, layered absorbent article assemblies have been developed that include a plurality of absorbent articles arranged in a stacked configuration. Each one of the stacked absorbent articles is removably attached to an adjacent absorbent article. The garment facing surface of the bottom-most one of the stacked absorbent articles is provided with an adhesive to secure the stacked absorbent article assembly to the undergarment of the user. During use, as the top-most (i.e. the body facing) one of the stacked articles becomes soiled the user may selectively remove the absorbent article to reveal a fresh absorbent article there under.

There are several problems associated with stacked absorbent article assemblies of the type described above. One common problem is that as the user removes a soiled absorbent article from the stack of absorbent articles the entire assembly detaches from the undergarment requiring the user to reattach the whole stacked absorbent article assembly to the undergarment. Another problem with stacked absorbent article assemblies of the type described above is that the adhesive that attaches one absorbent article to an adjacent absorbent article is too strong making the removal of one absorbent article from an adjacent absorbent article difficult.

Some conventional stacked absorbent article assemblies attempt to secure one absorbent article to an adjacent article by providing a seal along only the outer peripheral edge of the article. A problem with this type of configuration is that, again, it is often difficult for the user to remove one article from an adjacent article. In addition, since the articles are not secured to one another within the boundary defined by the sealed peripheral edge, the articles may tend to bunch during use. Further, if a mechanical bond is used to adhere the absorbent articles to one another, the mechanical bond may cause leak through of fluid from one absorbent article to the next, thereby destroying the cleanliness of the underlying absorbent article.

Another problem associated with conventional stacked absorbent article assemblies is that as a soiled absorbent article is removed from the stack, the adhesive located on the bottom surface thereof may leave an adhesive residue on the top surface of the underlying absorbent article. This residue can cause the exposed absorbent article to have a sticky feeling against the skin of the user thereby causing discomfort.

In view of the above it is an object of the present invention to provide a stacked absorbent article assembly that overcomes the shortcomings of the prior art stacked absorbent article assemblies described above.

### SUMMARY OF THE INVENTION

In view of the foregoing the present invention provides, according to a first aspect of the invention, a stacked absorbent article assembly including at least a first and second absorbent articles arranged in a stacked configuration, an adhesive arranged between the first and second absorbent articles for selectively securing the first and second absorbent articles, and wherein the
200 radians per second residing in a quadrangle ABCD wherein the quadrangle is defined
of the adhesiv
about 0.015 and 0.4 respectively at a frequency of about 200 radians per second.

### BRIEF DESCRIPTION OF THE DRAWINGS

Examples of embodiments of the present invention will now be described with reference to the drawings, in which:
Fig. 1 is a perspective view of a stacked absorbent article assembly in accordance with a first embodiment of the present invention, showing the top absorbent article thereof partially removed from the bottom absorbent article thereof;
Fig. 2 is an exploded perspective view of the stacked absorbent article assembly shown in Fig. 1 showing the individual layers of the absorbent articles;
Fig. 3 is sectional view of the top absorbent article shown in Fig. 1;
Fig. 4 is a sectional view of the bottom absorbent article shown in Fig 1;
Fig. 5 is a perspective view of a stacked absorbent article assembly in accordance with a second embodiment of the present invention;
Fig. 6 is an exploded perspective view of the stacked absorbent article assembly shown in Fig. 5 showing the individual layers of the absorbent articles;
Fig. 7 is a perspective view of a stacked absorbent article assembly in accordance with a third embodiment of the present invention;
Fig. 8 is an exploded perspective view of the stacked absorbent article assembly shown in Fig. 7;
Fig. 9 is a graphical plot of G' and G" versus temperature of an adhesive suitable for use as the inter-article adhesive in a stacked absorbent article assembly according to the present invention, showing the crossover temperature T_{c} of the adhesive;
Fig. 10 is a bar graph indicating the crossover temperature T_{c} of adhesives suitable for use as the inter-article adhesive in stacked absorbent article assemblies according to the present invention, as well as the crossover temperature T_{c} of comparative adhesives;
Fig 11 is a graphical plot of G' versus shear rate from 1 rad/sec to 125 rad/sec of adhesives suitable for use as the inter-article adhesive in stacked absorbent article assemblies according to the present invention, as well as a graphical plot of G' of comparative adhesives;
Fig 12 is a bar graph indicating the [G'₃₅ at 100 rad*sec⁻¹]/[G'₃₅ at 1 rad*sec⁻¹] value of adhesives suitable for use as the inter-article adhesive in stacked absorbent article assemblies according to the present invention, as well as the [G'₃₅ at 100 rad*sec⁻¹]/[G'₃₅ at 1 rad*sec⁻¹] value of comparative adhesives;
Fig 13 is a graphical plot of G" versus shear rate from 1 rad/sec to 125 rad/sec of adhesives suitable for use as the inter-article adhesive in stacked absorbent article assemblies according to the present invention, as well as a graphical plot of G" of comparative adhesives;
Fig. 14 is a bar graph indicating the [G"₃₅ at 100 rad*sec⁻¹]/[G"₃₅ at 1 rad*sec⁻¹] value of adhesives suitable for use as the inter-article adhesive in stacked absorbent article assemblies according to the present invention, as well as the [G"₃₅ at 100 rad*sec⁻¹]/[G"₃₅ at 1 rad*sec⁻¹] value of comparative adhesives; and
   of adhesives suitable for use as the inter-article adhesive in stacked absorbent article assemblies according to the present invention, as well as comparative adhesives, illustrating the quadrangle ABCD.

### DETAILED DESCRIPTION OF THE INVENTION

The present invention relates to a stacked absorbent article assembly that includes at least a first and second absorbent article arranged in a stacked configuration. As used herein, the term absorbent article shall mean disposable absorbent articles, such as, pantiliners, sanitary napkins, interlabial devices, adult incontinence devices, and diapers. These articles typically have a fluid permeable body-facing side and fluid impermeable garment facing side and may include an absorbent core arranged there between. Additional layers such as a transfer layer, distribution layer, acquisition layer, etc. may also be included.

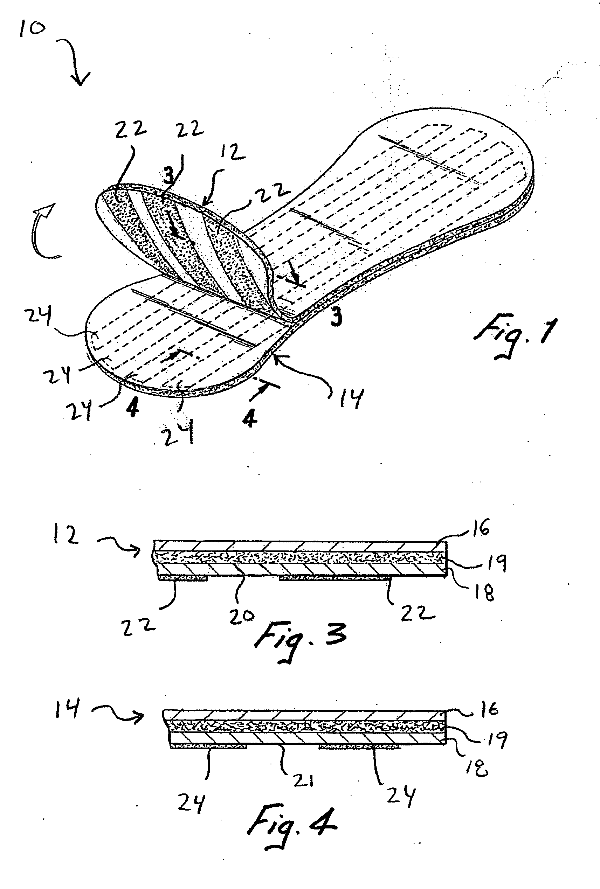

Fig. 1 is a perspective view of a stacked absorbent article assembly 10 in accordance with a first embodiment of the present invention. In the embodiment of the invention shown in Fig. 1 the stacked absorbent article assembly includes a top absorbent article 12 and a bottom absorbent article 14. As shown in Fig. 1, the top absorbent article 12 and bottom absorbent article 14 are arranged in a stacked configuration. As discussed in further detail below, the top absorbent article 12 and bottom absorbent article 14 are "selectively secured" to one another such that they are securely held together yet at the same time a user may selectively remove the top absorbent article 12 from the bottom absorbent article when the top absorbent article 12 becomes soiled. In this manner, as the top absorbent article 12 becomes soiled the user may manually remove the same to reveal the clean bottom absorbent article 14 there under.

As shown in Fig. 2 and Fig. 3 the top absorbent article 12, i.e. the upper-most absorbent article in the stacked absorbent article assembly 10, includes a liquid permeable cover 16, a liquid impermeable barrier 18, and an optional absorbent core 19 arranged between the cover 16 and the barrier 18. The liquid impermeable barrier 18 includes, on a bottom surface 20 thereof, an inter-article adhesive 22 that functions to "selectively secure" the top absorbent article 12 to the bottom absorbent article 14. That is, the adhesive 22 functions to securely adhere the top article 12 to the bottom article 14 when the stacked absorbent article assembly 10 is arranged in a user's panty but at the same time permits the user to manually remove the top article 12 from the bottom article 14 when the top absorbent article 12 becomes soiled.

In one embodiment of the invention, the inter-article adhesive 22 is applied to a bottom surface 20 of the barrier 18 such that it covers about 40% to about 70% of said bottom surface 20. The inter-article adhesive 22 is applied to the bottom surface 20 in the amount of from about 8 gsm (g/m²) to about 25 gsm, or in another embodiment of the invention, from about 8 gsm to about 12 gsm.

In order to securely hold the top absorbent article 12 and the bottom absorbent article 14 together, the inter-article adhesive 22 must have an adjacent absorbent article attachment force (AAF) of greater than about 5 grams per inch (g/in). The method for measuring the adjacent absorbent article attachment force (AAF) is set forth in detail herein. The inter-article adhesive 22 has an adjacent absorbent article attachment force (AAF) in one embodiment of the invention in the range of about 10 g/in to about 150 g/in and in another embodiment of the invention in the range of about 10 g/in to about 25 g/in.

As show in Fig. 2 and 4 the bottom absorbent article 14, i.e. the bottom-most absorbent article in the stacked absorbent article assembly 10, includes a liquid permeable cover 16, a liquid impermeable barrier 18, and an optional absorbent core 19 arranged between the cover 16 and the barrier 18. Preferably, the liquid permeable cover 16, liquid impermeable barrier 18, and absorbent core 19 of the bottom absorbent article 14 are formed from the same materials as those of top absorbent article 12. Liquid impermeable barrier 18 of bottom absorbent article 14 includes a garment facing surface 21 which is provided with a garment attachment adhesive 24. The garment attachment adhesive 24 initially functions to secure the stacked absorbent article assembly 10 to the undergarment. Upon removal of the top absorbent article 12, the garment attachment adhesive 24 functions to secure the bottom absorbent article 14 to the undergarment.

In order to securely attach the stacked absorbent article assembly 10 to an undergarment during use, the garment attachment adhesive 24 must have a garment attachment force (GAF) greater than about 30 g/in and less than about 450 g/in. The method for measuring garment attachment force (GAF) is set forth in detail herein. In one embodiment of the invention the garment attachment adhesive 24 has a garment attachment force (GAF) in the range from about 100 g/in to about 400 g/in, and in another embodiment of the invention between about 200 g/in and about 300 g/in.

In one embodiment of the invention, the garment attachment adhesive 24 is applied to the garment facing surface 21 of the bottom absorbent article 14 such that it covers from about 30% to about 70% of the garment facing surface 21 and in another embodiment it covers from about 45% to about 60% of the garment facing surface 21. The garment attachment adhesive 24 is applied to the garment facing surface 21 in one embodiment of the invention in an amount from about 10 gsm to about 40 gsm and in another embodiment from about 20 gsm to about 35 gsm.

In order to enable the user to easily remove the top absorbent article 12 from the bottom absorbent article 14, without inadvertently removing the entire absorbent article assembly 10 from the undergarment, the adjacent article attachment force (AAF), in the first embodiment of the invention, must be less than one-third the garment attachment force (GAF). At the same time, the adjacent article attachment force (AAF) must be sufficient to securely retain the top absorbent article 12 to the bottom absorbent article 14. Thus, according to the first embodiment of the invention, the adjacent article attachment force (AAF) is greater than 5 g/in and less then 1/3 the garment attachment force (GAF).

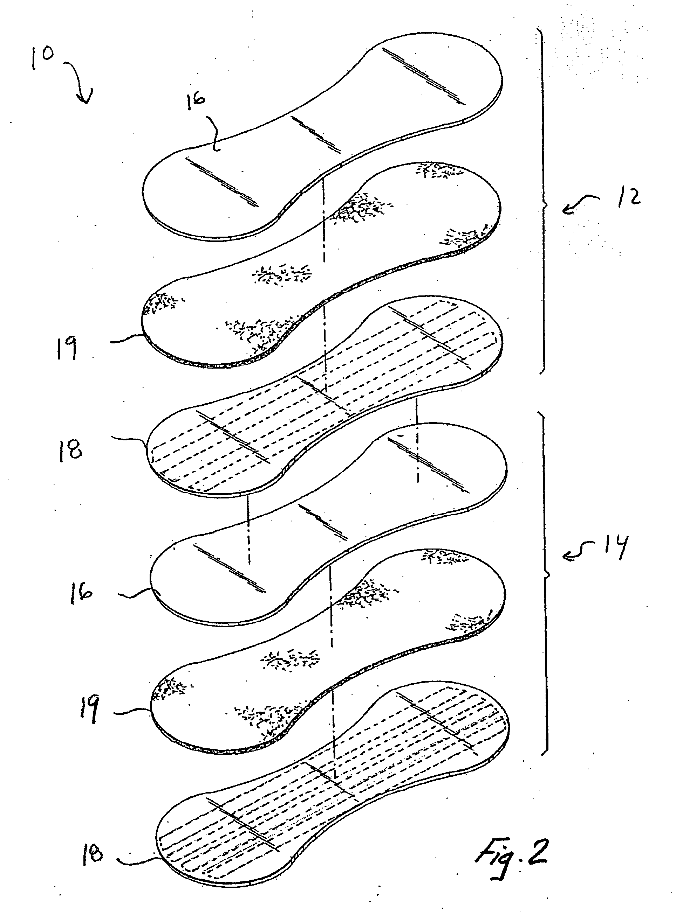

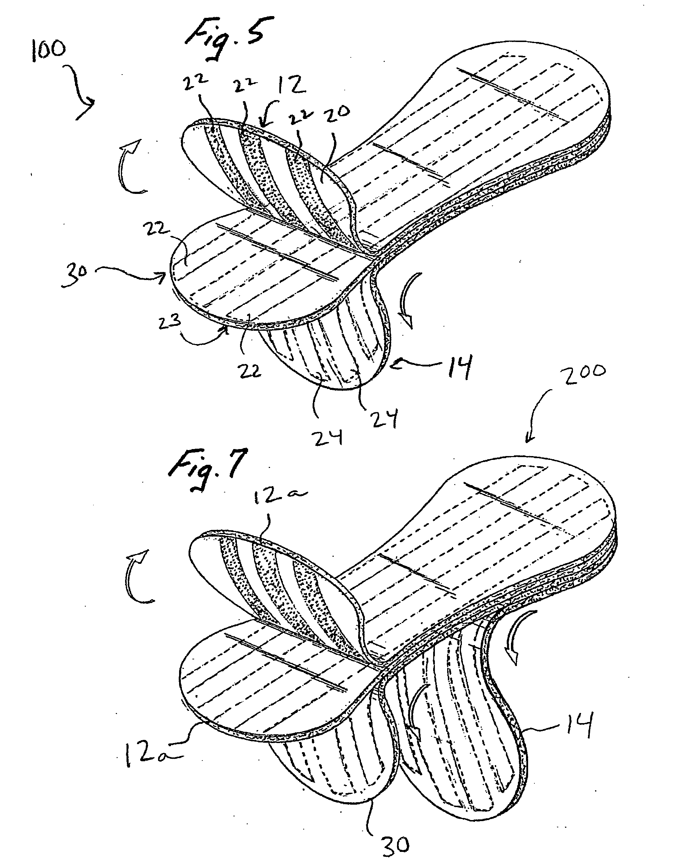

Figs. 5 and 6 show a stacked absorbent article assembly 100 in accordance with a second embodiment of the present invention. In the embodiment of the invention shown in Figs. 5 and 6 the stacked absorbent article assembly 100 includes a top absorbent article 12, a bottom absorbent article 14, and an intermediate absorbent article 30 arranged between the top absorbent article 12 and the bottom absorbent article 14. The absorbent articles 12, 14 and 30 are selectively secured to one another such that the top absorbent article 12 is manually removable from the intermediate absorbent article 30. Likewise, once the top absorbent article 12 is removed from the intermediate absorbent article 30, the intermediate absorbent article 30 is manually removable from the bottom absorbent article 14. In this manner, as the top absorbent article 12 becomes soiled the user may remove the same the reveal clean the intermediate article 30 there under. Likewise, after the intermediate absorbent article 30 becomes soiled the user may remove the intermediate absorbent article 30 from the bottom absorbent article 14 to thereby revel the clean bottom absorbent article 14.

The top absorbent 12 and bottom absorbent article 14 in the second embodiment 100 are essentially identical in construction to the corresponding structures described above with respect to the first embodiment. However, in the second embodiment 100, the inter-article adhesive 22 located on a bottom surface 20 of the top absorbent article 12, functions to selectively secure the top absorbent article 12 to the intermediate absorbent article 30.

In the stacked absorbent article assembly 100, the bottom absorbent article 14 includes on a garment facing surface 21 thereof, a garment attachment adhesive 24. The garment attachment adhesive is selected and applied in amount such that it has a garment attachment force (GAF) greater than about 30 g/in and less than about 450 g/in.

In the stacked absorbent article assembly 100, the inter-article adhesive 22 located on the bottom surface 20 of the top absorbent article 12 is selected and applied in an amount such that it has an adjacent article attachment force (AAF₂) that is less than or equal to 1/12 (GAF) and greater than 5 g/in. The adjacent article attachment force (AAF₂) between the top absorbent article 12 and the intermediate absorbent article 30 is selected in this range to insure that top absorbent article 12 is securely adhered to the intermediate absorbent article 30 yet at the same time can easily be removed by the user when soiled.

The intermediate absorbent article 30, in one embodiment of the invention, is essentially identical in construction to the first absorbent article 12 and the bottom absorbent article 14. That is, the intermediate absorbent article 30 includes a liquid permeable cover 16, a liquid impermeable barrier 18, and an optional absorbent core 19 arranged between the cover 16 and the barrier 18. The liquid impermeable barrier 18 of the intermediate absorbent article 30 includes, on a bottom surface 23 thereof, an inter-article adhesive 22 that functions to "selectively secure" the intermediate absorbent article 30 to the bottom absorbent article 14.

In the stacked absorbent article assembly 100, the inter-article adhesive 22 located on the bottom surface 23 of the intermediate absorbent article 30 is selected and applied in an amount such that it has an adjacent article attachment force (AAF₁) that is less than 1/3 (GAF) and greater than 15 g/in. The adjacent article attachment force (AAF₁) between the intermediate absorbent article 30 and the bottom absorbent article 14 is selected in this range to insure that the intermediate absorbent article 30 is securely attached to the bottom absorbent article 14 yet also can also be easily removed from the bottom absorbent article 14 by the user when soiled.

To insure that the removal of the top absorbent 12 does not result in the inadvertent simultaneously removal of the intermediate absorbent article 30, the adjacent article attachment force (AAF₁) between the intermediate absorbent article 30 and the bottom absorbent article 14 should be at least 10 g/in greater than the adjacent article attachment force (AAF₂) between the top absorbent article 12 and the intermediate absorbent article 30. Stated another way, in the second embodiment of the invention 100, AAF₁ - AAF2 > 10 g/in.

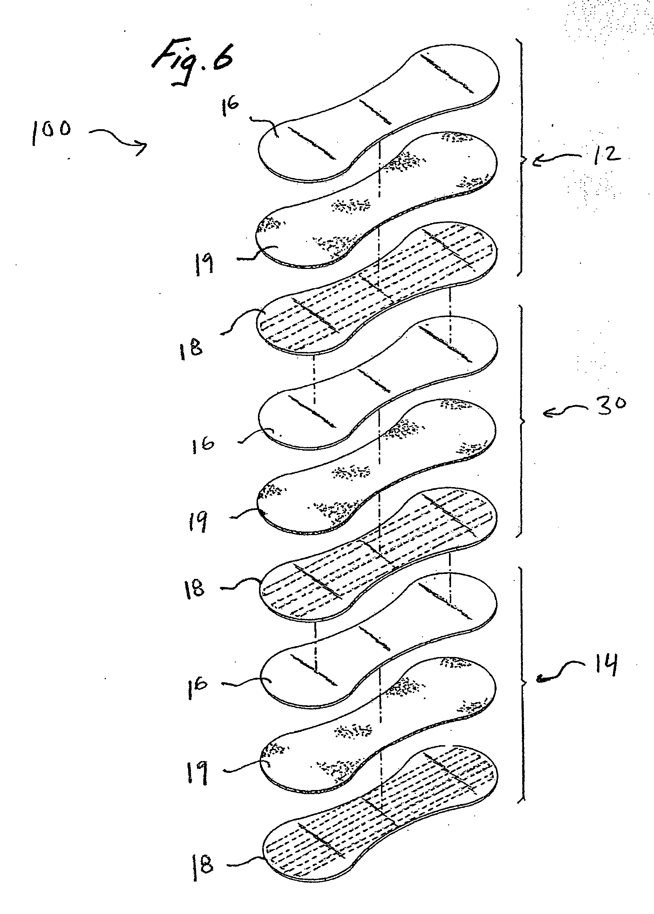

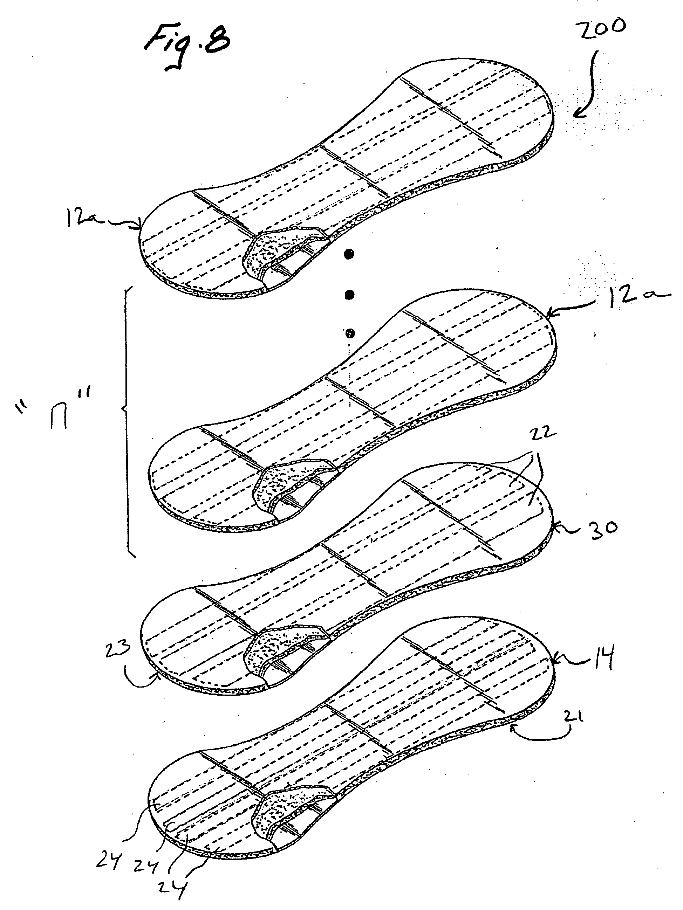

Figs. 7 and 8 show a stacked absorbent article assembly 200 in accordance with a third embodiment of the present invention. In the embodiment of the invention shown in Figs. 7 and 8 the stacked absorbent article assembly 200 includes a bottom absorbent article 14, an intermediate absorbent article 30 arranged in abutting face to face relationship with the bottom absorbent article 14, and a plurality ("n") of absorbent articles 12a arranged in a stacked configuration on top of the intermediate absorbent article 30. Thus, according to the embodiment of the invention shown in Figs. 7 and 8, the stacked absorbent article assembly 200 includes four or more absorbent articles. The absorbent articles 12a, 30 and 14 in the third embodiment generally have the same construction as the corresponding structures discussed above with respect to the first and second embodiments. The absorbent articles 12a in the third embodiment essentially have the same structure as the absorbent article 12 discussed above in reference to the first and second embodiments of the invention.

In the stacked absorbent article assembly 200, the bottom absorbent article 14 includes, on a garment facing surface 21 thereof, a garment attachment adhesive 24. The garment attachment adhesive 24 is selected and applied in amount such that it has a garment attachment force (GAF) greater than 60 g/in and less than 450 g/in.

In the stacked absorbent article assembly 200, the inter-article adhesive 22 located on the bottom surface 23 of the intermediate absorbent article 30 is selected and applied in an amount such that it has an adjacent article attachment force (AAF) that is less than 1/3 (GAF) and greater than 15 g/in. The adjacent article attachment force (AAF) between the intermediate absorbent article 30 and the bottom absorbent article 14 is selected in this range to insure that the intermediate absorbent article 30 is securely attached to the bottom absorbent article 14 yet also can also be easily removed from the bottom absorbent article 14 by the user when soiled.

Each of the plurality of articles 12a is selectively secured to an adjacent absorbent article. The bottom-most article 12a is selectively secured to the intermediate absorbent article 30 and each of the remaining articles 12a is selectively secured to an underlying adjacent article 12a. As one moves upward in the absorbent article assembly 200, i.e. upward from the intermediate absorbent article 30, the adjacent article attachment force (AAF) for each article 12a is less than or equal to twenty-five percent of the preceding adjacent article attachment force. For example, assume the absorbent article assembly 200 includes four absorbent articles, a bottom absorbent article 14, an intermediate absorbent article 30 arranged in abutting relationship to the bottom absorbent article 14, and two absorbent articles 12a sequentially arranged in a stacked configuration on top of the intermediate absorbent article 30. Assume the bottom absorbent article 14 includes, on a garment facing surface 21 thereof, a garment attachment adhesive 24, that is selected and applied in amount such that it has a garment attachment force (GAF) of 400 g/in. Assume the inter-article adhesive 22 located on the bottom surface 23 of the intermediate absorbent article 30 is selected and applied in an amount such that it has an adjacent article attachment force (AAF) that is less than 1/3 (GAF) and greater than 15 g/in, for example 100 g/in. The absorbent article 12a arranged in abutting relationship to the intermediate absorbent article 300 would then have an adjacent article attachment force (AAF) of less than or equal to 25 g/in. The absorbent article 12a arranged on top of the first absorbent article 12a would then have an adjacent article attachment force (AAF) of less than or equal to 6.25 g/in.

Thus, according to the third embodiment of the invention 200, the adjacent article attachment force (AAF) for a particular absorbent article 12a can be expressed as follows:
AAF
In the above formula AAF is the adjacent article attachment force for the particular article 12a and AAFa is the adjacent article attachment force between the two preceding articles in the stacked absorbent article assembly 200.

### Cover Layer

The liquid permeable cover layer 16 may be a relatively low density, bulky, high-loft non-woven web material. The cover layer 16 may be composed of only one type of fiber, such as polyester or polypropylene or it may include a mixture of more than one fiber. The cover layer 16 may be composed of bi-component or conjugate fibers having a low melting point component and a high melting point component. The fibers may be selected from a variety of natural and synthetic materials such as nylon, polyester, rayon (in combination with other fibers), cotton, acrylic fiber and the like and combinations thereof. Preferably, the cover layer 16 has a basis weight in the range of about 10 gsm to about 75 gsm.

Bi-component fibers may be made up of a polyester layer and a an polyethylene sheath. The use of appropriate bi-component materials results in a fusible non-woven fabric. Examples of such fusible fabrics are described in U.S. Pat. No. 4,555,430 issued Nov. 26, 1985 to Chicopee. Using a fusible fabric increases the ease with which the cover layer may be mounted to the absorbent layer and/or to the barrier layer. The cover layer 16 preferably has a relatively high degree of wettability, although the individual fibers comprising the cover may not be particularly hydrophilic. The cover layer 16 should also contain a great number of relatively large pores. This is because the cover layer 16 is intended to take-up body fluid rapidly and transport it away from the body and the point of deposition. Therefore, the cover layer 16 contributes little to the time taken for the napkin to absorb a given quantity of liquid (penetration time). Advantageously, the fibers, which make up the cover layer 16 should not lose their physical properties when they are wetted, in other words they should not collapse or lose their resiliency when subjected to water or body fluid. The cover layer may be treated to allow fluid to pass through it readily. The cover layer also functions to transfer the fluid quickly to the other layers of the absorbent system. Thus, the cover layer is advantageously wettable, hydrophilic and porous. When composed of synthetic hydrophobic fibers such as polyester or bi-component fibers, the cover layer may be treated with a surfactant to impart the desired degree of wettability. The cover layer 16 material may be formed using any number of known nonwoven manufacturing techniques, such as spunlacing, spunbonding, latex bonding and the like.

According to one specific embodiment of the present invention, the cover layer 16 is a spunbond material having a basis weight of 17 gsm, product code 17W21B2SA, commercially available from CNC International Co., Ltd., Rayong, Thailand .

Alternatively, the cover layer 16 can also be made of polymer film having large pores. Because of such high porosity, the film accomplishes the function of quickly transferring body fluid to the inner layers of the absorbent system. The cover layer 16 may be embossed to the remainder of the absorbent core 19 in order to aid in promoting hydrophilicity by fusing the cover 16 to the next layer. Such fusion may be effected locally, at a plurality of sites or over the entire contact surface of cover layer 16 and absorbent core 19. Alternatively, the cover layer may be attached to the absorbent core 19 by other means such as by adhesion.

### Absorbent Core

The absorbent core 19 may comprise a single layer of material or may comprise multiple layers. In one embodiment, the absorbent core is a blend or mixture of cellulosic fibers and superabsorbent disposed in and amongst fibers of that pulp. Cellulosic fibers that can be used in the absorbent core are well known in the art and include wood pulp, cotton, flax and peat moss. Wood pulp is preferred. Pulps can be obtained from mechanical or chemi-mechanical, sulfite, kraft, pulping reject materials, organic solvent pulps, etc. Both softwood and hardwood species are useful. Softwood pulps are preferred. It is not necessary to treat cellulosic fibers with chemical debonding agents, cross-linking agents and the like for use in the present material. Some portion of the pulp may be chemically treated as discussed in U.S. Pat. No. 5,916,670 to improved flexibility of the product. Flexibility of the material may also be improved by mechanically working the material or tenderizing the material.

The absorbent core 19 can contain any superabsorbent polymer (SAP), which SAPs are well known in the art. For the purposes of the present invention, the term "superabsorbent polymer" (or "SAP") refers to materials which are capable of absorbing and retaining at least about 10 times their weight in body fluids under a 0.5 psi pressure. The superabsorbent polymer particles of the invention may be inorganic or organic crosslinked hydrophilic polymers, such as polyvinyl alcohols, polyethylene oxides, crosslinked starches, guar gum, xanthan gum, and the like. The particles may be in the form of a powder, grains, granules, or fibers. Preferred superabsorbent polymer particles for use in the present invention are crosslinked polyacrylates, such as the product offered by Sumitomo Seika Chemicals Co., Ltd. Of Osaka, Japan, under the designation of SA70N and products offered by Stockhausen Inc.

In one embodiment of the invention, the core 19 is a hot through air nonwoven material having a basis weight of 75 gsm, product code FD51526, commercially available from Beijing Da Yuan, Beijing, China.

Absorbent core materials for use in the present invention preferably have a basis weight in the range of about 10 gsm to about 400 gsm.

It is possible that the absorbent core 19 could be integrated with the cover and/or barrier such that there is essentially only a single layer structure or a two layer structure including the function of the multiple layers described herein.

### Barrier Layer

Underlying the absorbent core is a barrier layer 18 comprising liquid-impervious film material so as to prevent liquid that is entrapped in the absorbent core 19 from egressing the sanitary napkin and staining the wearer's undergarment. The barrier layer 18 is preferably made of polymeric film, although it may be made of liquid impervious, air-permeable material such as repellent-treated non-woven or micropore films or foams. The barrier layer may be breathable, i.e., permits vapor to transpire. Known materials for this purpose include nonwoven materials and microporous films in which microporosity is created by, inter alia, stretching an oriented film. Single or multiple layers of permeable films, fabrics, melt-blown materials, and combinations thereof that provide a tortuous path, and/or whose surface characteristics provide a liquid surface repellent to the penetration of liquids may also be used to provide a breathable backsheet. The cover layer 16 and the barrier layer 18 are joined along their marginal portions so as to form an enclosure or flange seal that maintains the absorbent layer 19 captive. The joint may be made by means of adhesives, heat- bonding, ultrasonic bonding, radio frequency sealing, mechanical crimping, and the like and combinations thereof.

Liquid impervious film materials suitable for use as the barrier layer 18 in the present invention include polyethylene and polypropylene films having a basis weight in the range from about 5 gsm to about 50 gsm. According to one specific embodiment of the present invention, the barrier layer 18 is a 22.5 gsm polyethylene film, product code CPE-72W (22.5), commercially available from Swanson Plastics PTE, Ltd., Singapore.

### Inter-Article Adhesive

The inter-article adhesives 22 employed in the stacked absorbent article assemblies according to the present invention are hot melt adhesives based on styrenic block copolymers, that is the adhesive formulations contain styrenic block copolymers, tackifying resins, and plasticizing oils. More specifically, the inter-article adhesives 22 employed herein are typically made of styrene-isoprene-styrene block copolymers (SIS) or styerene-ethylene-butylene block copolymers (SEBS).

Suitable block copolymers for use in the invention include linear or radial co-polymer structures having the formula (A-B)x wherein block A is a polyvinylarene block, block B is a poly(monoalkenyl) block, x denotes the number of polymeric arms, and wherein x is an integer greater than or equal to one. Suitable block A polyvinylarenes include, but are not limited to Polystyrene, Polyalpha- methylstyrene, Polyvinyltoluene, and combinations thereof. Suitable Block B poly(monoalkenyl) blocks include, but are not limited to conjugated diene elastomers such as for example polybutadiene or polyisoprene or hydrogenated elastomers such as ethylene butylene or ethylene propylene or polyisobutylene, or combinations thereof. Commercial examples of these types of block copolymers include Kraton elastomers from Kraton Polymers LLC, Vector elastomers from Dexco polymers, Stereon from Firestone Tire & Rubber Co. & SIBStar Polymers from Kaneka Co. Ltd.

Suitable tackifying resins include aliphatic petroleum resins and the hydrogenated derivatives thereof; aromatic petroleum resin and the hydrogenated derivatives thereof; and aliphatic or aromatic petroleum resins and the hydrogenated derivatives thereof, and combinations thereof; natural and modified resins; glycerol and pentaerythritol esters of natural and modified resins; polyterpene resins; copolymers and terpolymers of natural terpenes; phenolic modified terpene resins and the hydrogenated derivatives thereof. Commercial examples of these types of resins include Escorez from Exxon Chemical aliphatic hydrocarbon and cycloaliphatic resins, Wingtacke from Goodyear Tire & Rubber Co. synthetic polyterpene resins including aromatic modified versions, Arkon partially and fully hydrogenated aromatic resins from Arakawa Chemicals,Foral hydrogenated rosin ester, Staybelite hydrogenated modified rosin, Poly-pale polymerized rosin, Permalyn rosin ester, Pentalyn rosin ester, Adtac oil extended hydrocarbon resin, Piccopale aromatic hydrocarbon, Piccotac, Hercotac aromatic modified aliphatic hydrocarbon, is Regalrez cycloaliphatic resins, or Piccolyte from Eastman Chemical Co., Zonatac styrenated terpene resin, Zonarez rosin ester and Zonester rosin ester from Arizona Chemical and Nevtac aromatic modified aliphatic hydrocarbon from Neville Chemical Company.

Specific inter-article adhesives 22 suitable for use in the present included HM-2703, HL-2268, and HL-2110X from HB Fuller Co. of St. Paul, Minnesota. In addition the construction adhesives NW-1023 from HB Fuller Co. and 34-5539, from National Starch and Chemical Co. of Bridgewater, NJ were also evaluated for comparative purposes. The pad attachment adhesive NW-1042, from HB Fuller Co. was also evaluated for comparative purposes. It is noted that NW-1023 from HB Fuller Co., 34-5539, from National Starch and Chemical Co. of Bridgewater, NJ and NW-1042, from HB Fuller Co. are not suitable for use as the inter-article adhesive 22 in stacked absorbent article assemblies according to the present invention.

It has been discovered that adhesives suitable for use as inter-article adhesives 22 in the present invention have certain rheolgical properties that enable the adhesive to securely adhere one absorbent article to the adjacent absorbent article while at the same time permitting the user to easily manually remove one absorbent article from the adjacent absorbent article to which it is attached. Further, the adhesives employed as inter-article adhesives 22 in the present invention have certain rheolgical properties that minimize the adhesive residue left on the top surface of an underlying absorbent article when the overlying absorbent article is removed therefrom. In this way the exposed top surface of the underlying article does not have a "sticky" feeling to the user during use.

Rheometer devices for determining rheolgocial properties of adhesives, and the techniques for using such devices, are well known to those skilled in the art. Further explanations of polymer rheology and their measurement are discussed in: Viscoelastic Properties of Polymers, John D. Ferry, John Wiley & Sons, third edition, pages 264-280 (1980); "Studies of Triblock Copolymer-Tackifying Resin Interactions by Viscoelasticity and Adhesive Performance", Mun Fu Tse, Journal of Adhesion Science Technology, Vol 3. No. 7, pages 551-570 (1989); and test procedure ASTM-D 4440-84 the disclosures of which are incorporated herein by reference and made a part hereof.

The rheological properties set forth herein were measured using a ARES Rheometer, manufactured by TA Instruments of Wilmington, Delaware. The adhesives described herein were tested in a parallel plate test geometry 25mm in diameter and approximately 2 mm thickness. Adhesive samples were subject to oscillatory shear and the instrument. The material was subjected to a sinusoidal strain and the stress response was measured, in order to determine the elastic and viscous material response simultaneously. For this type of procedure, a motor was used to apply a sinusoidal strain to an adhesive sample, in shear, and the resulting stress was measured with a force transducer. The rheological material behavior was measured as a function of, temperature, and frequency (shear rate).

To measure adhesive rheological response as a function of temperature, adhesives were tested at a constant shear rate of 10 radians per second, from 0 to 130°C. The elastic (storage) shear modulus (G') and the viscous (loss) shear modulus (G") were determined based on the adhesives stress response. The loss tangent (G"/G'), or Tan was also determined. Characterization of these materials as a function of temperature provides information as to the application range for a given material as a function of temperature. The temperature at which viscous flow properties become the dominate rheological property as defined by the G'/G" crossover point, which will be referred to herein as the T_{c} was also determined.

To measure adhesive response as a function of shear rate, a constant temperature of 35°C was used, in order to simulate a relevant application temperature, and samples were subject to shear rates from 0.1 radians per second to 125 radians per second. The elastic (storage) shear modulus (G') and the viscous (loss) shear modulus (G") were determined based on the adhesives stress response. The loss tangent, or Tan d, was also determined (G"/G'). The low shear rate portion of the test range provided rates that more closely simulate static conditions as observed in use, such as products may see during storage and shipping, or sedentary conditions during wear. The shear rates in the moderate to higher ranges more closely simulate rates the adhesive may see during removal from use or under process and lamination conditions.

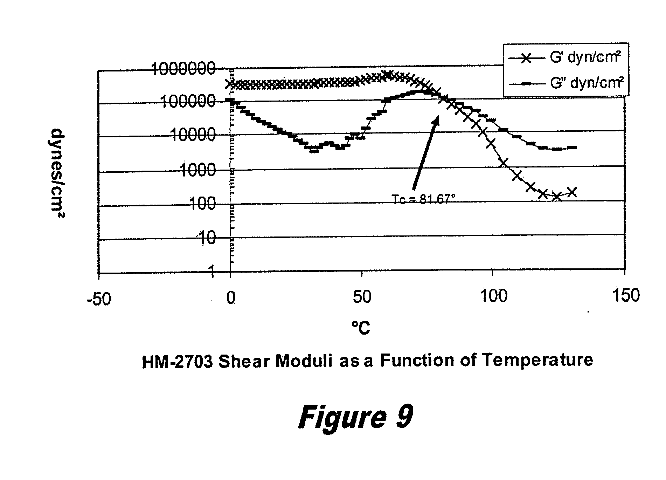

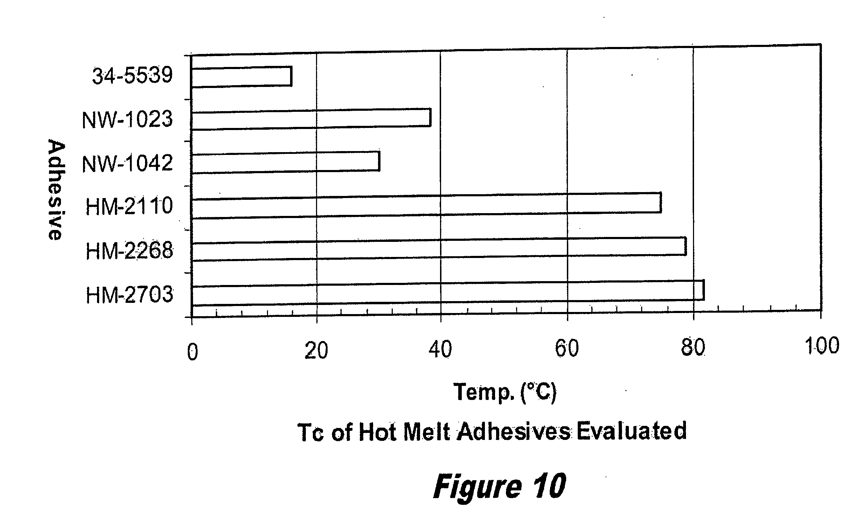

Reference is made to Fig. 9 which shows a plot of G' and G" versus temperature from 0° C to 130° C for an adhesive suitable for use as the inter-article adhesive according to the present invention, in particular HM-2703, commercially available from HB Fuller Co. of St. Paul, Minnesota. As shown, HM-2703 has a crossover temperature T_{c} of 81.67° C. Fig. 10 is a bar graph indicating the crossover temperature T_{c} of adhesives suitable for use as the inter-article adhesive in stacked absorbent article assemblies according to the present invention, namely HM-2703, HL-2268, and HL-2110X from HB Fuller Co. of St. Paul, Minnesota. Fig. 10 also provides the crossover temperature T_{c} for comparative example adhesives NW-1023 from HB Fuller Co., 34-5539, from National Starch and Chemical Co. and NW-1042, from HB Fuller Co..

Based upon the rheological analysis described herein it has been determined that adhesives useful as inter-article adhesives 22 in stacked absorbent article assemblies according to the present invention have a G'/G" crossover temperature T_{c} of greater than 50° C. That is, adhesives employed in stacked absorbent articles according to the present invention have a G'/G" temperature that can be represented by the following formula: ${T}_{c} > 50 ⁢ ° C$

In one embodiment of the invention, adhesives employed in stacked absorbent articles according to the present invention have crossover temperature in the range between about 70° C and 90° C.

Without being bound by theory, it is believed that adhesives having a crossover temperature T_{c} in the above identified range can be easily processed and applied to the article during manufacture but at the same time minimize the adhesive residue left on the top surface of an underlying absorbent article when the overlying absorbent article is removed therefrom.

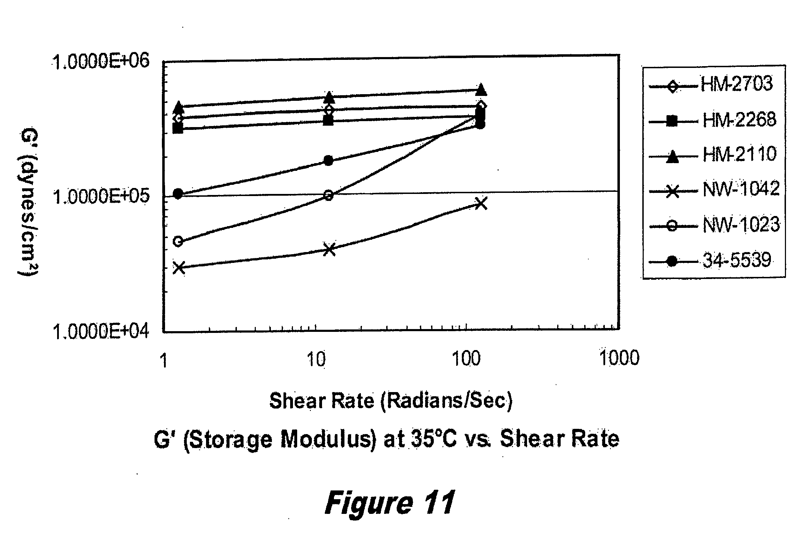

Reference is made to Fig 11 which shows a graphical plot of G' versus shear rate from 1 rad/sec to 125 rad/sec of adhesives suitable for use as the inter-article adhesive in stacked absorbent article assemblies according to the present invention. Suitable inter-article adhesives include HM-2703, HL-2268, and HL-2110X from HB Fuller Co. of St. Paul, Minnesota. Fig. 11 also provides G' versus shear rate plot for comparative example adhesives NW-1023 from HB Fuller Co., 34-5539, from National Starch and Chemical Co. and NW-1042, from HB Fuller Co..

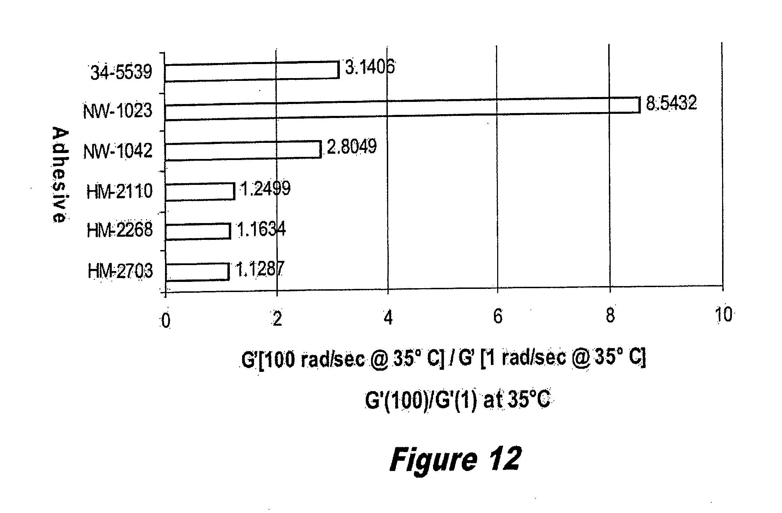

Fig 12 is a bar graph indicating the [G'₃₅ at 100 rad*sec⁻¹]/[G'₃₅ at 1 rad*sec⁻¹] value of adhesives suitable for use as the inter-article adhesive in stacked absorbent article assemblies according to the present invention, as well as the [G'₃₅ at 100 rad*sec⁻¹]/[G'₃₅ at 1 rad*sec⁻¹] value of comparative adhesives

Based upon the rheological analysis described herein it has been determined that adhesives useful as inter-article adhesives 22 in stacked absorbent article assemblies according to the present invention have an elastic (storage) shear modulus (G') at 100 rad/sec, at 35° C, relative to an elastic shear modulus (G') at 1 rad/sec, at 35° C, of < 2.5. This relationship can be represented by the following formula: $\left[{Gʹ}_{35} at 100 rad*{sec}^{- 1}\right] / \left[{Gʹ}_{35} at 1 rad*{sec}^{- 1}\right] < 2.5$
In one embodiment of the invention the [G'₃₅ at 100 rad*sec⁻¹]/[G'₃₅ at 1 rad*sec⁻¹] ratio is in the range of between about 0.5 to about 1.5.

Without being bound by theory, it is believed that adhesives having a [G'₃₅ at 100 rad*sec⁻¹]/[G'₃₅ at 1 rad*sec⁻¹] ratio in the above recited range securely retain the absorbent articles in a stacked configuration during manufacture and use but at the same time enable the user to easily remove one absorbent article from the adjacent absorbent article when the absorbent article becomes soiled.

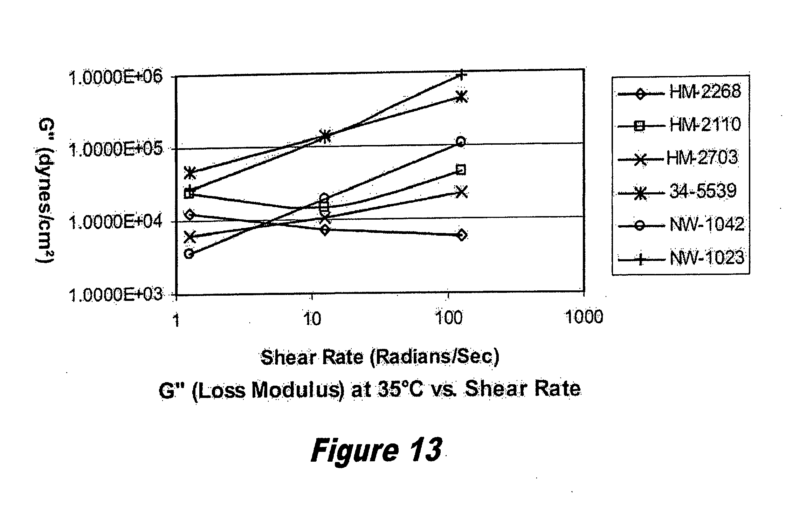

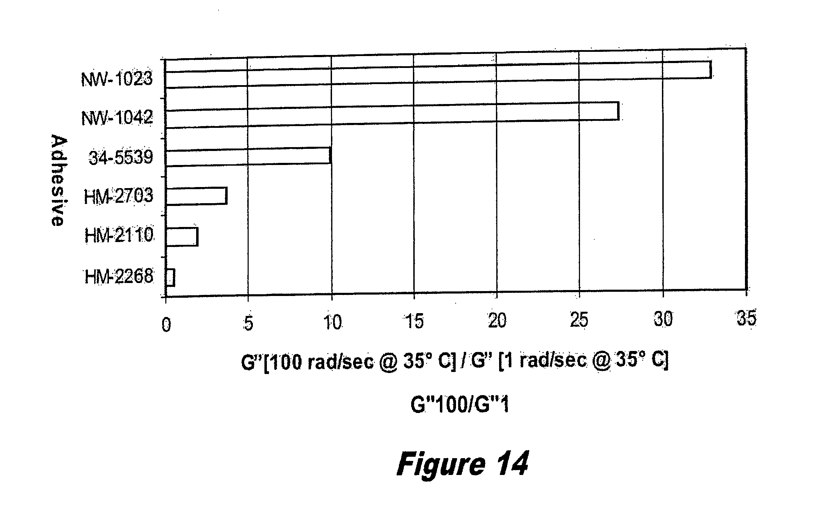

Fig 13 is a graphical plot of G" versus shear rate from 1 rad/sec to 125 rad/sec of adhesives suitable for use as the inter-article adhesive in stacked absorbent article assemblies according to the present invention, as well as a graphical plot of G" of comparative adhesives. Fig. 14 is a bar graph indicating the [G"₃₅ at 100 rad*sec⁻¹]/[G"₃₅ at 1 rad*sec⁻¹] value of adhesives suitable for use as the inter-article adhesive in stacked absorbent article assemblies according to the present invention, as well as the [G"₃₅ at 100 rad*sec⁻¹]/[G"₃₅ at 1 rad*sec⁻¹] value of comparative adhesives.

Based upon the rheological analysis described herein it has been determined that adhesives useful as inter-article adhesives 22 in stacked absorbent article assemblies according to the present invention are characterized by a ratio of viscous (loss) shear modulus (G") at 100 rad/sec, at 35° C, relative to a viscous (loss) shear modulus (G") at 1 rad/sec, at 35° C, of < 24. This relationship can be represented by the following formula: $\left[{Gʺ}_{35} at 100 rad*{sec}^{- 1}\right] / \left[{Gʺ}_{35} at 1 rad*{sec}^{- 1}\right] < 24$
In one embodiment of the invention the [G"₃₅ at 100 rad*sec⁻¹]/[G"₃₅ at 1 rad*sec⁻¹] ratio is in the range of between about 0.5 to about 5.0.

Without being bound by theory, it is believed that adhesives having a [G"₃₅ at 100 rad*sec⁻¹]/[G"₃₅ at 1 rad*sec⁻¹] ratio in the above recited range securely retain the absorbent articles in a stacked configuration during manufacture and use but at the same time enable the user to easily remove one absorbent article from the adjacent absorbent article when the absorbent article becomes soiled.
ec of adhesives suitable for use as the inter-article adhesive in stacked absorbent article assemblies according to the present invention, as well as comparative adhesives, illustrating the quadrangle ABCD.

Based upon the rheological analysis described herein it has been determined that adhesives useful as inter-article adhesives 22 in stacked absorbent article assemblies to 1.1 and more preferably 0.015 to 0.25 at frequency of 0.2 radians per second and a Tan at a frequency of 200 radians per second.

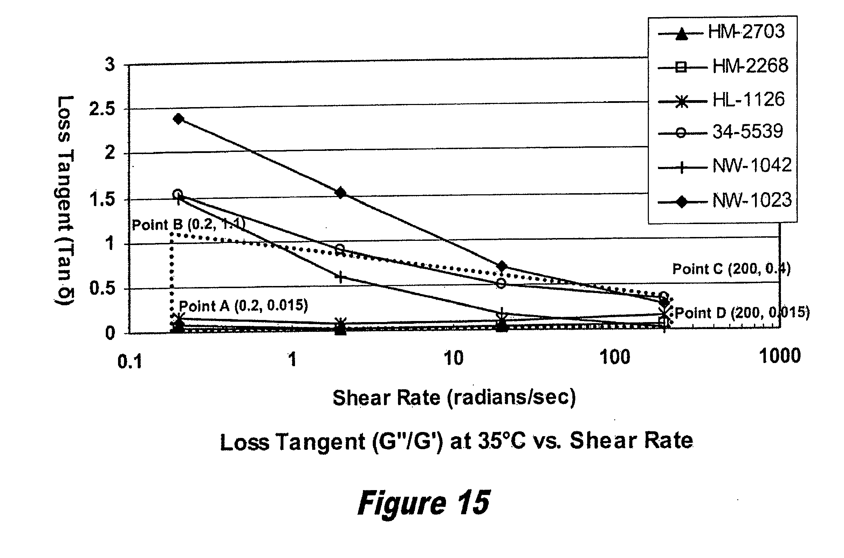

Preferred adhesives useful as inter-article adhesives 22 in stacked absorbent a
ABCD between the frequency range of about 0.2 radians per second to about 200 radians per second, as seen in Fig. 15. The sides of Quadrangle ABCD are determined by pl
and upper frequencies, i.e. 0.2 radians per second and 200 radians per second.
within the quadrangle ABCD provide sufficient adhesion to effectively adhere retain the absorbent articles in a stacked configuration during manufacture and use but at the same time enable the user to easily remove one absorbent article from the adjacent absorbent ar
outside the quadrangle ABCD provide insufficient adhesion to keep the stacked absorbent articles laminated to one another or in the alternative result in the top absorbent article destructively compromising the cover of the bottom absorbent article when the user attempts to remove the top absorbent article. For example, 34-5539 and NW-1023
t in which the top absorbent article cannot be removed from the bottom absorbent article without causing destructive failure of the bottom absorbent article.

### Garment-Attachment Adhesive

The garment attachment adhesive 24 may comprise a pressure sensitive adhesive that is applied as strips, swirls, or waves, and the like. As used herein, the term pressure-sensitive adhesive refers to any releasable adhesive or releasable tenacious means. Suitable adhesive compositions, include, for example, water-based pressure-sensitive adhesives such as acrylate adhesives. Alternatively, the adhesive composition may include adhesives based on the following: emulsion or solvent-borne adhesives of natural or synthetic polyisoprene, styrene-butadiene, or polyacrylate, vinyl acetate copolymer or combinations thereof; hot melt adhesives based on suitable block copoylmers - suitable block copolymers for use in the invention include linear or radial co-polymer structures having the formula (A-B)x wherein block A is a polyvinylarene block, block B is a poly(monoalkenyl) block, x denotes the number of polymeric arms, and wherein x is an integer greater than or equal to one. Suitable block A polyvinylarenes include, but are not limited to Polystyrene, Polyalpha-methylstyrene, Polyvinyltoluene, and combinations thereof. Suitable Block B poly(monoalkenyl) blocks include, but are not limited to conjugated diene elastomers such as for example polybutadiene or polyisoprene or hydrogenated elastomers such as ethylene butylene or ethylene propylene or polyisobutylene, or combinations thereof. Commercial examples of these types of block copolymers include KratonTM elastomers from Shell Chemical Company, VectorTM elastomers from Dexco, SolpreneTM from Enichem Elastomers and StereonTM from Firestone Tire & Rubber Co.; hot melt adhesive based on olefin polymers and copolymers where in the olefin polymer is a terpolymer of ethylene and a co-monomers, such as vinyl acetate, acrylic acid, methacrylic acid, ethyl acrylate, methyl acrylate, n-butyl acrylate vinyl silane or maleic anhydride. Commercial examples of these types of polymers include Ateva( polymers from AT plastics), Nucrel( polymers from DuPont), Escor (from Exxon Chemical).

Commercially available adhesives useful as the garment attachment adhesive 24 include NW-1042 from HB Fuller Co., St. Paul, Minnestoa and Sanicare 8060 from Henkel Adhesives, Dusseldorf, Germany.

In one embodiment of the invention, the garment attachment adhesive 24 is applied to a garment facing surface of the stacked absorbent article assembly such that it covers from about 30% to about 70% of the garment facing surface and in another embodiment it covers from about 45% to about 60% of the garment facing surface. The garment attachment adhesive 24 is applied to the garment facing surface of the stacked absorbent article assembly in one embodiment of the invention in an amount from about 10 gsm to about 40 gsm and in another embodiment in the amount from about 20 gsm to about 35 gsm.

### Test Methods for Determining Adjacent Article Attachment Force (AAF) and Garment Attachment Force (GAF)

The adjacent article attachment force (AAF) between two adjacent absorbent articles of a stacked absorbent article assembly according to the present invention is determined by measuring the peel force between the respective articles. For the garment attachment force (GAF), the peel force required to remove the bottom article of the assembly from a cloth substrate is measured. The peel force is determined by a modified ASTM D 3330 PEEL ADHESION OF PRESSURE SENSITIVE-TAPE test as described below. In general, according to ASTM D 3330 , a pressure-sensitive adhesive laminate sample is peeled at a selected angle (typically 90 degrees or 180 degrees) and at a selected speed (from 12-1200 inches per minute). An electronic load cell measures the peel/release force, then feeds the information to a data acquisition unit. Movable reed switches are positioned along the test bed and determine the portion of the test sample data to be recorded by the data acquisition unit. The data acquisition unit collects the selected portion of test data from the load cell and stores these data points in memory for use in calculating the maximum, minimum and average values. This data can then be downloaded through a RS232 connection port, to an appropriate receiving program such as EZStats on a Personal computer.

For the adjacent article attachment force (AAF) test, rather than evaluating the adherence of a single-coated tape (Test Method A, ASTM D 3330) to a standard steel panel, or to another surface of interest, when peeled at a 180 degree angle and at a specific rate, the modified test method as set forth herein evaluates the removal of an absorbent article from an underlying absorbent article to which it is attached. The top absorbent article is like the single-coated tape and the bottom absorbent article is like the surface of interest from which it is being removed. For the garment attachment force (GAF), the bottom most absorbent article of the assembly is placed on the apparatus and removed from a standard bleached cotton fabric, 80 x 80 count, antistatic (Antron 3), commercially available from Test Fabrics Inc., P.O. Box 53, 200 Blackford Ave., Middlesex, N.J.

### APPARATUS

The apparatus necessary for the evaluation of the adjacent article attachment force (AAF) and the garment attachment force (GAF) is the ChemInstruments Adhesion/Release Tester AR-1000 (ChemInstruments, 510 Commercial Drive, Fairfield, Ohio 45014), having the following parts:
Power switch - turns on/off the power
Function switches - control the start and stop of data acquisition
Test sled - provides a surface to hold the test material
Clutch handle - engages and disengages the test sled with the drive chain
Load cell - measures the forces involved with an Adhesion/Release test
Load cell assembly - consists of the mounting bracket for the load cell with grip
Grip - secures the free end of the test strip to the load cell
Mast - hold the pivoting bracket with load cell assembly and permits running tests at angles between 90 degrees and 180 degrees. A small peg on the backside of the mast allows for convenient positioning of the load cell for testing at a 180 degree angle.

The apparatus necessary for sample preparation is a 1-inch specimen precision cutter. The specimen cutter shall hold two single-edge razor blades in parallel planes, a precise 1-inch distance apart, to form a cutter of exact specimen widths (JDC Precision Sample Cutter, Thwing-Albert Instrument Company, Philadelphia, PA).

### NUMBER AND PREPARATION OF SPECIMENS

In order to perform the procedure for this test, as explained below, five representative stacked absorbent article assemblies are necessary to determine the adjacent article attachment force (AAF). An additional, identically constructed, five representative stacked absorbent article are necessary to determine the garment attachment force (GAF). The samples are tested after at least 24 hours after the production of the absorbent articles and conditioning the absorbent articles by leaving them at rest at least 2 hours in the temperature and humidity controlled test room (23± 1 degree C temperature, 50% ± 2% relative humidity). All tests should be conducted under these conditions as well. Using the precision cutter the absorbent articles are cut in the longitudinal direction and in the center of the absorbent article. Samples that are slightly skewed will alter the accuracy of the results; thus, it is critical that the samples are cut precisely in the longitudinal direction along the centerline of the product. All samples should be one inch in width by six and half inches in length in order to be able to obtain a six-inch peel evaluation and to have at least half an inch of the sample to insert into the peel tester clasp or grip. If the original product is not a full six and half inches in length then all peel evaluation should be performed throughout the length of the product and averaged across the corresponding distance. A one-inch wide sample should still be used.

### PROCEDURE - ADJACENT ARTICLE ATTACHMENT FORCE (AAF)

The procedure for determining the adjacent article attachment force (AAF) is as follows:

If the stacked absorbent article assembly to be analyzed includes more than two absorbent articles, the particular adjacent article force to tested is selected. If any absorbent articles are located above the two articles to be tested then these articles should be first removed. For example, in a three absorbent article assembly, if the adjacent article attachment force to be tested is between the intermediate and bottom article, then the top absorbent article should be removed from the assembly. In a three article assembly if the adjacent article force to tested is between the top and intermediate absorbent articles then no articles need be removed since there are no articles located above the two articles being tested. For a two article stacked absorbent article assembly no absorbent article should be removed from the assembly since there is only a single adjacent article attachment force in such an assembly.

All testing apparatus must be calibrated according to the operating manual and prior to beginning any testing. All testing and calibration is to be completed using a 1000 gram load cell and with a 180 degree peel setup. For a 180-degree peel test, the test sled and the load cell must be in the horizontal position, with the load cell assembly at the lowest position on the mast. Remove the release paper (release liner) from the bottom absorbent article of the stacked absorbent article assembly and place the stacked absorbent article assembly in the middle of the test sled with the garment attachment adhesive facing the sled. If the garment attachment means is something other than adhesive, first cover 100% of the test sled with double-coated tape (Scotch, Double-Coated Tape, 665) and then place the stacked absorbent article assembly on the center of the test sled The stacked absorbent article assembly should be placed on the test sled with the longest length edge being parallel to the longest length side on the test slide. Gently separate the two absorbent articles between which the adjacent article force is to be evaluated until approximately half an inch of the upper most absorbent article is free. Using a one-inch by three-inch wide piece of masking tape (Scotch Performance Masking Tape, 2380), tape down the articles underlying the upper most absorbent article to the test sled in the perpendicular direction. Using another three inch by one inch piece of masking tape create a pull-tab for the top absorbent article so you can comfortably insert it into the grip by folding over the tape over the ends of the top absorbent article.

Turn the clutch handle on the test sled to disengage it from the drive chain and move the sled to the start position at the right end of the test bed. Bend the free end of the test strip with the masking tape pull-tab back over the end of the test strip and insert it into the grip. Note the remaining length of the product and adjust the start and stop function switches accordingly and/or determine through which distance the average peel force is to be determined. Set the test sled speed to be a minimum of 12 inches/minute. Make sure the AR-1000 is in the Run Menu. Engage the test sled by rotating the clutch handle clockwise. Once, the test sled has moved past the Stop switch, the average value of the test data collected will be displayed. Record this value. To run additional tests, replace the test material and repeat the procedure.

The above process is repeated for five specimens and an average is calculated to provide the relevant adjacent article attachment force (AAF).

### PROCEDURE - GARMENT ATTACHMENT FORCE (GAF)

The procedure for determining the garment attachment force (GAF) is as follows. All testing apparatus must be calibrated according to the operating manual and prior to beginning any testing. All testing and calibration is to be completed using a 1000 gram load cell and with a 180 degree peel setup. For a 180-degree peel test, the test sled and the load cell must be in the horizontal position, with the load cell assembly at the lowest position on the mast. Remove the bottom absorbent article from the stacked absorbent article assembly. Remove the release paper (release liner) from the bottom absorbent article and place the bottom absorbent article (adhesive side down or garment attachment means down) onto a piece of standard bleached cotton (80 x 80 count, antistatic (Antron 3), supplied by: Test Fabrics Inc., P.O. Box 53, 200 Blackford Ave., Middlesex, N.J). Use a 4.5-pound roller to apply the absorbent article to the cotton. Move the roller the length of the bottom absorbent article, one pass in each direction. The bottom absorbent article and cotton should be placed on the test sled with the longest length edge being parallel to the longest length side on the test slide. Gently separate the front edge of the bottom absorbent article from the cotton and use a three inch by one inch piece of masking tape create a pull-tab for the bottom absorbent article so you can comfortably insert it into the grip by folding over the tape over the ends of the absorbent article.

Turn the clutch handle on the test sled to disengage it from the drive chain and move the sled to the start position at the right end of the test bed. Bend the free end of the test strip with the masking tape pull-tab back over the end of the test strip and insert it into the grip. Note the remaining length of the product and adjust the start and stop function switches accordingly and/or determine through which distance the average peel force is to be determined. Set the test sled speed to be a minimum of 12 inches/minute. Make sure the AR-1000 is in the Run Menu. Engage the test sled by rotating the clutch handle clockwise. Once, the test sled has moved past the Stop switch, the average value of the test data collected will be displayed. Record this value. To run additional tests, replace the test material and repeat the procedure.

The above process is repeated for five specimens and an average is calculated to provide the garment attachment force (GAF).

### EXAMPLE

A stacked absorbent article assembly including a top and bottom absorbent article was constructed as described below.

In order to produce the bottom absorbent article a nonwoven cover material ( 17 gsm spunbond cover, product code 17W21B2SA, commercially available from CNC International Co., Ltd., Rayong, Thailand) is unwound and the non-body facing side is continuously slot coated with adhesive (product code NS34-5539-UV, commercially available from National Starch, Shanghai, China) with 6.0 gsm of adhesive. The absorbent core (75.0 gsm hot through air bonded core, product code FD51526, commercially available from Beijing Da Yuan, Beijing, China) is unwound. The absorbent core material is arranged such that the smooth side thereof is arranged in abutment with the barrier while the rough side thereof is arranged in abutment with the cover. A barrier film (a 22.5 gsm polyethylene film material, product code CPE-72W (22.5), commercially available from Swanson Plastics PTE, Ltd., Singapore) is unwound and the internal surface thereof is continuously spray coated with construction adhesive. The barrier film is then combined with the cover/absorbent core web. The continuous web is then crimp (heat) sealed on the periphery and cut into the specified dimensions of the product. The product area of the bottom absorbent product is 6897 square mm. The trim waste is removed via vacuum. A release film with silicone coating (product code FL-40, commercially available from Sopal, France) is unwound and simultaneously, 27.5 gsm of positioning adhesive (product code NW 1042 commercially available from H.B. Fuller, Guangzhou, China) is applied in a longitudinal strip pattern along the full length of the product. The garment attachment adhesive covers approximately 56% of the product. The bottom absorbent article is then placed to the side until the top absorbent layers are produced.

In order to produce the top absorbent article the nonwoven cover roll (CNC, 17 gsm spunbond cover) is unwound and the non-body facing side is continuously slot coated with adhesive (National Starch, NS34-5539-UV) with 6.0 gsm of adhesive. The absorbent core (Beijing Da Yuan, FD51526) is unwound. The smooth side of the absorbent core will be faced to the barrier while the rough side of the absorbent core is faced to the cover. The cover is combined with the unwound absorbent core. The laminated web is then embossed and the barrier layer ( a 22.5 gsm polyethylene film material, Swanson Plastics PTE) is unwound. An internal surface of the barrier film is continuously spray coated with construction adhesive. The barrier film is then combined with the cover/absorbent core web. The layers are then crimp (heat) sealed on the periphery and cut into the specified dimensions of the product. The product area of the top absorbent article is 6897 square mm. The trim waste is removed via vacuum. A release film with silicone coating (product code FL-40, commercially available from Sopal, France) is unwound and simultaneously, 10 gsm of inter-article adhesive (product code HM 2703, commercially available from HB Fuller Co. of St. Paul, Minnesota) is applied in a longitudinal strip pattern along the full length of the product at a temperature of 325 degrees Fahrenheit and minimum open time before being nipped to the barrier (milliseconds). The inter-article adhesive covers approximately 60% of the product. The top absorbent article is then ready to be laminated together to the bottom absorbent article.

In order to laminate the top absorbent article to the bottom absorbent article the release film is removed from the top absorbent article exposing the inter-article adhesive. The top absorbent article is then placed directly on top of the bottom absorbent article. Since the products have identical shapes and surface areas they should be in perfect alignment with each other. The articles are then laminated together by inserting them cover facing upward in the longitudinal direction into the ChemInstruments HL-101 Hot Roll Laminator. The samples are fed through the hot roll laminator at a speed of 4.4 feet per minute and a gap of loose ten thousands/tight eleven thousands. The temperature of the hot roll laminator is maintained at 225 degrees Celsius and the pressure is set to be 60 psi. The articles are then gently placed between two pieces of release paper and immediately after being removed from the hot roll laminator are squeezed together using a 5-pound roller to ensure full contact of the adhesive. The roller is run the full length of the absorbent article, one pass in each direction. The articles are then gently placed aside to cool.

For the above described stacked absorbent article assembly, adjacent article attachment force (AAF) between the top and bottom absorbent article was measured to be 13.88 g/in and the garment attachment force (GAF) was measured to be 269.24 g/in.

From the foregoing description, one skilled in the art can ascertain the essential characteristics of this invention, and without departing from the spirit and scope thereof, can make various changes and modifications. Embodiments set forth by way of illustration are not intended as limitations on the variations possible in practicing the present invention.

## Claims

1. A stacked absorbent article assembly comprising:
at least a first and second absorbent articles arranged in a stacked configuration;
an adhesive arranged between said first and second absorbent articles for selectively securing said first and second absorbent articles; and
radians per second residing in a quadrangle ABCD wherein said quadrangle is defined by
respectively at a frequenc
about 0.015 and 0.4 respectively at a frequency of about 200 radians per second.

2. The stacked absorbent article assembly according to claim 1, wherein said adhesive
y of 0.2 radians per second to 200 radians per second residing in a quadrangle ABCD wherein said quadrangle is defined by graphically plotting frequency
ABCD having po
at a frequency of about 200 radians per second.

3. The stacked absorbent article according to claim 1, wherein said adhesive has a crossover temperature T_{c} > 50°.

4. The stacked absorbent article assembly according to claim 3, wherein adhesive has a crossover temperature in the range of from about 70° C to about 90° C.

5. The stacked absorbent article assembly according to claim 1, wherein said adhesive has a G'_{[100 rad/sec @ 35° C]} G' _{[1 rad/sec @ 35° C]} ratio of less than 2.5.

6. The stacked absorbent article assembly according to claim 5, wherein said adhesive has a G'_{[100 rad/sec @ 35° C]} / G' [1 _{rad/sec@35°C]} ratio of between about 0.5 to about 1.5.

7. The stacked absorbent article assembly according to claim 1, wherein said adhesive has a G"_{[100 rad/sec @ 35° C]} G" _{[1rad/sec @ 35° C]} ratio of less than 24.0.

8. The stacked absorbent article assembly according to claim 7, wherein said adhesive has a G"_{[100rad/sec@35°C]} /G" _{[1 d/sec@35°C]} ratio of between about 0.5 and about 5.0.

9. A stacked absorbent article assembly comprising:
at least a first and second absorbent articles arranged in a stacked configuration;
an adhesive arranged between said first and second absorbent articles for selectively securing said first and second absorbent articles;
radians per second residing in a quadrangle ABCD wherein said quadrangle is defined by
said adhesive, said quadrangle ABCD having points A and
about 0.015 and 0.25 respectively at a frequency of about 200 radians per second;
wherein adhesive has a crossover temperature in the range of from about 70° C to about 90° C;
wherein said adhesive has a G'_{[100 rad/sec @ 35° C]} / G' _{[1 rad/sec @ 35° C]} ratio of between about 0.5 to about 1.5; and
wherein said adhesive has a G"_{[100 rad/sec @ 35° C]} / G" _{[1 rad/sec @ 35° C]} ratio of between about 0.5 and about 5.0.
